# EUROPÄISCHE PATENTSCHRIFT

(11) **EP 2 706 929 B1**
(45) Veröffentlichungstag und Bekanntmachung des Hinweises auf die Patenterteilung: **27.09.2017**
(21) Anmeldenummer: 12726581.7
(22) Anmeldetag: 14.05.2012
(51) Int. Cl.: A61B 17/221, A61F 2/90

(54) **THROMBEKTOMIEVORRICHTUNG**
THROMBECTOMY DEVICE
DISPOSITIF DE THROMBECTOMIE

(30) Priorität: 13.05.2011 DE 102011101522
(43) Veröffentlichungstag der Anmeldung: 19.03.2014
(73) Patentinhaber: Phenox GmbH, 44801 Bochum (DE)
(72) Erfinder: MONSTADT, Hermann, 44797 Bochum (DE); HANNES, Ralf, 44137 Dortmund (DE); ASCHERFELD, Jörg, 45529 Hattingen (DE)
(74) Vertreter: Thiel, Christian
(86) Internationale Anmeldenummer: PCT/EP2012/002060
(87) Internationale Veröffentlichungsnummer: WO 2012/156069

(56) Entgegenhaltungen:
- WO-A1-99/48440
- WO-A1-2004/008991
- US-A1- 2009 292 297
- US-A1- 2011 009 875

## Beschreibung

Die Erfindung betrifft eine Thrombektomievorrichtung mit einer im Wesentlichen zylindrischen Stentstruktur, die eine Vielzahl von Maschen aufweist sowie zwei Verbinder, die an verschiedenen Maschen am proximalen Ende der Stentstruktur angeordnet sind und einem Führungsdraht, der ein Kopplungselement aufweist, an das die Verbinder angekoppelt sind. Die Thrombektomievorrichtung ist insbesondere dafür bestimmt, Thromben im zerebralen Bereich, wie sie häufig bei Schlaganfällen zu finden sind, für den Patienten schonend und zuverlässig zu entfernen.

Thromboembolische Erkrankungen wie Herzinfarkt, Lungenembolie, periphere Thrombose, Organembolien, etc. werden typischerweise durch einen Thromboembolus (im Folgenden kurz: Thrombus), also einem viskoelastischen Blutklumpen aus Blutplättchen, Fibrinogen, Gerinnungsfaktoren etc., ausgelöst, der sich in einem Blutgefäß festgesetzt hat und diese ganz oder teilweise verschließt. Der Verschluss von Organarterien führt dabei zu einer Unterbrechung der Versorgung des abhängigen Gewebes mit Sauerstoff und Nährstoffen. Der Störung des Funktionsstoffswechsels mit Funktionsverlust folgt innerhalb kurzer Zeit das Erliegen des Strukturstoffwechsels mit dem Untergang des betroffenen Gewebes (Infarkt). Die häufigsten hiervon beim Menschen betroffenen Organe sind das Herz und das Gehirn. Solche Veränderungen betreffen aber auch die Extremitätenarterien und die Lungenarterien. Venöse Thrombosen und thromboembolische Verschlüsse kommen auch gehäuft in den Bein- und Beckenvenen vor. Das Krankheitsbild eines thrombotischen Verschlusses eines intrakraniellen Sinus kann durch die Störung der venösen Drainage des Hirngewebes zu schweren Hirnblutungen führen.

Angesichts der Schwere der durch Thromboembolien ausgelösten Krankheitsbilder und der Häufigkeit dieser Erkrankungen sind verschiedene Techniken zur Auflösung oder Entfernung von Thromben bekannt.

So ist es bekannt, solche Patienten mit thrombolytischen Mitteln wie Streptokinase oder Urukinase oder mit Antikoagulantien zu behandeln, was der Thrombolyse oder der Eindämmung des Thrombenwachstums dient. Da diese Behandlungsmethoden meist zeitintensiv sind, werden sie oftmals mit Methoden kombiniert, die der medizinischen Zerkleinerung oder Entfernung des Thrombus bzw. Embolus dienen.

Neben offenen chirurgischen Eingriffen kommen im Stand der Technik zunehmend transluminale bzw. endovaskuläre Katheter-geführte interventionelle Therapieformen zum Einsatz, da diese weniger invasiv sind. So ist es bekannt, den Thrombus mittels Unterdruck erzeugenden Saugkathetern oder mechanisch mit Kathetern, welche mit Fangkörben, Wendeln, Haken oder dergleichen versehen sind, aus dem Körper des Patienten zu entfernen, siehe US 6,245,089 B1; US 5,171,233 A1, Thomas E. Meier et al., Stroke 2002 (9) 2232.

Der Nachteil thrombolytischer Behandlungsmethoden liegt darin, das sie nach Ablauf des Zeitfensters nur noch selten Erfolg haben. Auch die bekannten transluminalen Vorrichtungen können einen Thrombus häufig nicht vollständig entfernen, wobei auch die Gefahr besteht, dass der Thrombus oder Fragmente davon freikommen und im Blutstrom zu kleinlumigeren Gefäßen verfrachtet werden, wo sie schwerer zu erreichen und zu behandeln sind. Des Weiteren eignen sich die im Stand der Technik bekannten Vorrichtungen aufgrund ihrer Dimensionen und/oder geringen Flexibilitäten nur ungenügend zur Entfernung von Thromben aus besonders kleinlumigen oder stark gewundenen Gefäßen, wie denen des Gehirns.

Die US 2011/009875 A1 offenbart eine Thrombektomievorrichtung mit einer im wesentlichen zylindrischen Stentstruktur, die eine Vielzahl von Maschen, zwei Verbinder an verschiedenen Maschen am proximalen Ende der Stentstruktur und einen Führungsdraht mit einem Kopplungselement zu den Verbindern aufweist.

Aus der WO 2004/008991 A1 ist ein medizinisches Implantat in Form eines offenen Stents bekannt, das zur Behandlung von Aneurysmen und anderen vaskulären Fehlbildungen bestimmt ist. Dieses Implantat wird mit Hilfe eines Führungsdrahts zum Einsatzort geführt und dort abgelöst. Es wurde vorgeschlagen, diese Kombination aus Implantat und Führungsdraht zur Extraktion von Thromben einzusetzen, wobei naturgemäß die Ablösung des Implantatteils vom Führungsdraht unterbleibt. Nachteil dieser Konstruktion aus Implantat und Führungsdraht ist allerdings eine relativ geringe Spann- oder Federkraft. Das Konstrukt entfaltet eine nicht immer ausreichende Scherwirkung auf den in der Gefäßwandung sitzenden Thrombus, so dass Reste im Gefäß verbleiben. Die Anbindung an den Führungsdraht über eine sich verjüngende Struktur (Träne) führt insbesondere zu einer Verschlankung des proximalen Bereichs der Struktur unter Zug, die der Effizienz des Konstrukts entgegensteht.

Angesichts der mit dem Stand der Technik verbundenen Nachteile besteht die Aufgabe der Erfindung in der Bereitstellung einer Vorrichtung zur Entfernung von Fremdkörpern und Thromben aus Blutgefäßen, welche insbesondere die Entfernung von Thromben aus kleinlumigen Gefäßen erlaubt und dabei über eine gute Manövrierfähigkeit in stark gewundenen Gefäßen aufweist und über eine große Wirkfläche verfügt.

Diese Aufgabe wird erfindungsgemäß mit einer Vorrichtung der eingangs genannten Art gelöst, die über einen sich wendelförmig über die Mantelfläche der Stentstruktur erstreckenden Schlitz verfügt, für welchen am proximalen Ende der Stentstruktur von einem Spannbügel überspannt ist.

Die erfindungsgemäße Vorrichtung besteht aus einer zylinderischen Struktur, wie sie auch Stents aufweisen, mit einer Vielzahl von Maschen. Sie ist über zwei Verbinder mit einem Führungsdraht verbunden, der die präzise Platzierung erlaubt. Die Verbinder sind am proximalen Ende in einer Maschenstruktur angeordnet und enden in einem Kopplungselement, das seinerseits das distale Ende des Führungsdrahts darstellt.

Der hier gebrauchte Begriff "proximal" bezeichnet die zum behandelnden Arzt weisende Seite, "distal" dagegen die vom Arzt wegweisende Seite, beispielsweise der Stentstruktur oder des Führungsdrahts.

Die Maschenstruktur des Stents kann eine geflochtene Struktur sein, d. h. aus einzelnen Drähten bestehen, ist aber vorzugsweise eine geschnittene Struktur, bei der aus einem Rohr geeigneten Durchmessers mit Hilfe eines Lasers die Maschenstruktur herausgeschnitten wird. Das Material ist in der Regel ein Metall, kann aber auch ein Kunststoff sein. Es muss über eine hinreichende Elastizität verfügen, die eine Kontraktion auf den Durchmesser eines üblichen Katheters erlaubt und andererseits bei der Freisetzung aus dem Katheter die Expansion auf den gewünschten und vorgegebenen Durchmesser.

Als Stentmaterialien kommen neben Eisenlegierungen (Edelstahl, Federstahl) und Kobalt-Chrom-Legierungen insbesondere Formgedächtnislegierungen in Frage, etwa binäre Nickel-Titan-Legierungen (Nitinol) und ternäre Nickel-Titan-Chrom-Legierungen (Chrom-dotierte Legierungen). Insbesondere Nitinol ist für die Anwendung in selbstexpandierenden Stentstrukturen im neurovaskulären Bereich bekannt.

Die erfindungsgemäße Vorrichtung ist im Prinzip eine flächige Struktur, die zu einem rohrförmigen Gebilde gerollt ist und einen Schlitz aufweist, der sich wendel- oder helixförmig über die Mantelfläche der Stentstruktur erstreckt. Dieser Schlitz kann dabei eine vollständige Wendel von 360° darstellen, aber auch eine nur partielle von beispielsweise etwa 180° oder 120°. Die Mantelfläche der Stentstruktur klafft im Bereich dieses Schlitzes auf, wobei die Breite des Schlitzes am Einsatzort jeweils auch vom Lumen des Gefäßes bestimmt wird, da sich die Stentstruktur nach der Freisetzung aus dem Katheter nur so weit entfalten kann, wie es das Gefäßlumen zulässt.

Um die Stentstruktur zum einen räumlich zu fixieren und andererseits mit einer gewissen Spannung zu versehen, erstreckt sich am proximalen Ende der Stentstruktur ein Spannbügel über den Schlitz. Der Spannbügel erhöht die Radialkraft der selbstexpandierenden Struktur, dient aber auch dazu, die einander gegenüberliegenden Kanten der Stentstruktur entlang des Schlitzes in ihrer Position zueinander festzuhalten.

Die erfindungsgemäße Thrombektomievorrichtung kann über dem proximalen Spannbügel hinaus weitere Spannbügel im zentralen und distalen Bereich aufweisen. Bei Verwendung von Formgedächtnismaterialien mit hinreichender Vorspannung kann aber auch auf jeglichen Spannbügel verzichtet werden.

Die erfindungsgemäße Thrombektomievorrichtung wird so eingesetzt, dass sie mittels eines Katheters an den Einsatzort verbracht wird und dort entweder im Thrombus selbst oder distal vom Thrombus freigesetzt wird. Die Vorrichtung expandiert im Gefäß und passt sich an das Gefäßlumen an. Entweder schon beim Aufspannen oder beim Zurückziehen verfängt sich das Thrombusmaterial in der Maschenstruktur und wird beim Zurückziehen der Vorrichtung in den Katheter mitgenommen. An der Gefäßwand anhaftende Teile des Thrombus werden durch die Scherwirkung der Maschen und der Kanten entlang des Schlitzes mitgenommen. Der Thrombus wird in den Katheter eingezogen und mit dem Katheter aus dem Körper entfernt.

Bei der Extraktion des Thrombus hat der wendelförmige Verlauf des Schlitzes über die Mantelfläche den besonderen Vorteil, dass die Kanten der Stentstruktur entlang des Schlitzes bei Zug tangential entlang des Umfanges der Gefäßwandung wandern. Dies verbessert die Scherwirkung. Gleichzeitig verbessert (vermindert) sich durch den wendelförmigen Verlauf die Biegesteifigkeit dergestalt, dass eine bessere Anpassung an kurvige Gefäße möglich ist. Dies erleichtert sowohl die Platzierung als auch die Extraktion von Thromben aus komplexen Gefäßstrukturen.

Der proximale Bügel verbessert den Radialkraftverlauf der Stentstruktur im proximalen Bereich. Insbesondere vermindert der Bügel eine Verschlankung der Stentstruktur und der Zugbelastung, wie sie beim Einziehen in den Katheter auftritt. Gleichzeitig wird eine zusätzliche Schälwirkung erreicht, wie sie auch von den Maschen und Kanten der Stentstruktur ausgeübt wird.

Von Bedeutung ist aber insbesondere die Verbesserung der Aufspannkraft im proximalen Bereich, die eine optimale Anpassung der Stentstruktur an das Gefäßlumen ermöglicht. Gleichzeitig werden die vom Schlitz voneinander getrennten Bereiche des Stents daran gehindert, sich gegeneinander zu verschieben.

Um ein problemloses Einziehen der Stentstruktur mit dem Bügel in den Katheter zu ermöglichen, weist der Spannbügel zum distalen Ende der Stentstruktur hin. Dies bedeutet, dass der Bogen des Bügels nach distal geschlossen ist, dagegen nach proximal zusammen mit den Verbindern eine Schlaufe bildet, die im Kopplungselement zusammenläuft, ähnlich der Öffnung eines Fangkorbs.

Alternativ überspannt der Spannbügel den Schlitz der Stentstruktur in einem wellenförmigen Verlauf, etwa dergestalt, dass der Bügel den Verlauf des Randes der Maschenstruktur auf einer Seite des Schlitzes aufnimmt und zur anderen Seite fortsetzt.

Gemäß einer Variante kann die erfindungsgemäße Stentstruktur am distalen Ende durch eine Maschenstruktur verschlossen sein, so dass sich thrombotisches Material darin wie in einem Fangkorb sammelt

Wie schon festgestellt, wird die erfindungsgemäße Stentstruktur vorzugsweise aus einem zylindrischen Rohr mit Hilfe eines Lasers geschnitten. Dies erlaubt es, den einzelnen Maschen einen besonderen Querschnitt zu verleihen, beispielsweise quadratisch, rechteckig oder trapezförmig. Bei den rechteckigen und trapezförmigen Ausführungsformen kann zum einen die schmale Seite des Querschnitts an der Außenfläche liegen, zum anderen die lange Seite. Bevorzugt ist es, dass die schmale Seite sowohl des Rechtecks wie insbesondere des Trapezes zur Gefäßwand weist, was ein leichteres Eindringen des Thrombus in die Maschenstruktur ermöglicht und die gute Verdrängung des Thrombusmasse bei der Expansion der Stentstruktur erlaubt.

Die am proximalen Ende der Stentstruktur angeordneten Verbinder führen von an den Schlitz angrenzenden proximalen Waben zu einem Kopplungselement, in dem sie zusammengeführt sind. Sie sind Teile der Stentstruktur und bestehen deshalb aus dem gleichen Material.

Der Führungsdraht der erfindungsgemäßen Thrombektomievorrichtung ist ein üblicher Führungsdraht, wie er für endovaskuläre und insbesondere neuroradiologische Zwecke eingesetzt wird. Er endet distal in dem Kopplungselement, das seinerseits die proximalen Enden der Verbinder aufnimmt.

Das Kopplungselement selbst kann ein einfacher Schweißpunkt sein, in dem Führungsdraht und Verbinder zusammengeführt sind. Es kann weiterhin aber auch ein übliches Kopplungselement sein, das die Freisetzung der zylindrischen Stentstruktur erlaubt, die insbesondere dann geboten ist, wenn eine Rückholung aus medizinischen Gründen nicht angezeigt ist, beispielsweise weil sie zu Schäden beim Patienten führen würde. In diesem Fall kann die Stentstruktur als Stent im Körper verbleiben und ihre Wirkung dadurch entfalten, dass sie im Thrombus einen Kanal ausbildet; der Thrombus wird durch die Maschenstruktur an die Gefäßwand gepresst.

Für letzteren Fall ist das Kopplungselement beispielsweise ein mechanisches Kopplungselement, das geeignet ist, bei Austritt aus dem Katheter die Verbinder freizusetzen. Zahlreiche solcher Systeme sind in der Fachliteratur beschrieben. Ebenfalls beschrieben sind hydraulische Ablösesysteme. Besonders geeignet sind elektrolytische Ablösungssysteme, bei denen ein elektrolytisch korrodierbares Teil durch Stromeinwirkung aufgelöst wird und die Verbindung zwischen Stentstruktur und Führungsdraht durchtrennt. Gemäß einer ersten Variante kann das Kopplungselement als solch ein elektrolytisch auflösbares Teil gestaltet sein, gemäß einer zweiten Variante sind die Verbinder mit einer solchen Ablösestelle bzw. einem separaten Ablöseelement versehen, das sich bei Stromeinwirkung auflöst. Geeignet als Ablöseelemente sind vorkorrodierte Edelstahlelemente, Magnesiumelemente oder Kobalt-Chrom-Legierungen. Solche Systeme sind in der Literatur beschrieben.

Bei der Gestaltung des proximalen Bereichs der zylindrischen Stentstruktur ist eine kurze Ausführung der Verbinder bevorzugt. Der Weg zwischen proximalem Ende der Maschenstruktur und Kopplungselement soll kurz gehalten werden. Dies verkürzt zum einen die ungenutzte Länge der Vorrichtung und erhöht andererseits die Spannung in der mit dem Spannbügel gebildeten Fangschlinge am proximalen Ende der Struktur.

Gemäß einer besonderen Ausführungsform kann der distale Bereich der zylindrischen Stentstruktur kegelförmig oder trompetenförmig aufgeweitet sein, um in diesem Bereich eine gute Anpassung an das Gefäßlumen zu ermöglichen. Bei der Extraktion von Thromben aus einem Gefäß kommt es auf einen möglichst großen Wirkbereich an, d. h. auf den Kontakt der Mantelfläche mit der Gefäßwand. Je größer die Kontaktfläche, desto größer die Chance, den Thrombus vollständig zu entfernen.

Führungsdraht und/oder Stentstruktur können in üblicher Weise mit Markern versehen sein, die röntgendicht sind, beispielsweise in Form von Spiralen oder Manschetten.

Die Erfindung wird durch die beiliegenden Abbildungen näher erläutert. Es zeigen:
- Figur 1: in flächiger Darstellung eine erste Variante der erfindungsgemäßen Stentstruktur;
- Figur 2: eine räumliche Darstellung der Stentstruktur von Figur 1;
- Figur 3: eine flächige Darstellung einer zweiten Variante einer erfindungsgemäßen Stentstruktur;
- Figur 4: eine räumliche Darstellung der Stentstruktur von Figur 3 mit angekoppeltem Führungsdraht;
- Figur 5: eine perspektivische Darstellung einer erfindungsgemäßen Stentstruktur mit zwei Verbindern;
- Figur 6: eine Darstellung der Stegquerschnitte der Stentstruktur;
- Figur 7: eine schematische Darstellung der erfindungsgemäßen Thrombektomievorrichtung,
- Figur 8: eine weitere Ausführungsform in flächiger Darstellung und
- Figur 9: eine räumliche Darstellung der Stentstruktur von Figur 8.

Figur 1 und 3 zeigen zwei Varianten einer erfindungsgemäßen zylindrischen Stentstruktur 1 mit den einzelnen Maschen 3 und 4 und den Verbindern 5 und 5'. Die Maschen 3 und 4 sind von zwei verschiedenen Typen, die einen (3) haben eine Wellenform, die anderen (4) eine bauchige Form mit zwei Spitzen. Im Zusammenwirken geben diese beiden Formen der Gesamtstruktur sowohl Stabilität als auch Flexibilität.

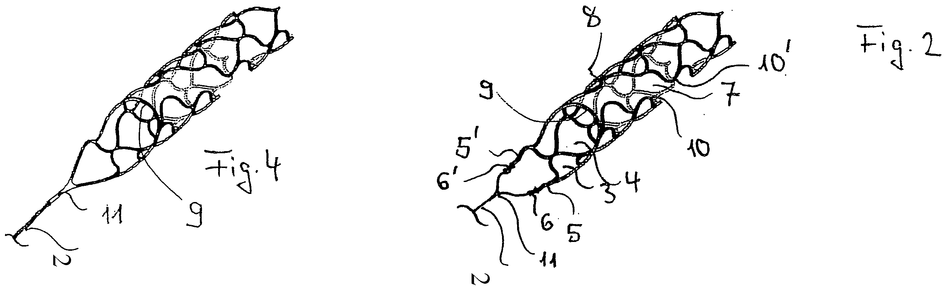

In der flächigen Darstellung der Abbildungen 1 und 3 verläuft durch die Stentstruktur ein Schlitz oder Kanal 7, der am proximalen Ende der Struktur von dem Spannbügel 9 überbrückt wird. Der Schlitz 7 wird begrenzt durch die Ränder 10 und 10' der Maschenstruktur. Der Schlitz 7 verläuft nicht parallel zur Längsachse der Struktur sondern schräg zur Längsachse, was sich in der räumlichen Darstellung als wendelförmiger Verlauf entlang der Mantelfläche (siehe Figur 2/4) darstellt.

Die Darstellung in Figur 1 und 3 ist eine flächige Darstellung der aufgeschnittenen Stentstruktur 1; die räumlichen Darstellungen sind in Figur 2 und 4 wiedergegeben. In der flächigen Darstellung grenzen die Maschen 3 unmittelbar an die Maschen 3' dergestalt, dass sich ein insgesamt rohrförmiges Gebilde mit in etwa halb um die Mantelfläche 8 umlaufendem Schlitz oder Kanal 7 ergibt.

Die Varianten von Figur 1 und 3 unterscheiden sich in der Form der Verbinder 5 und 5', die im Falle von Figur 3 länger ausgebildet sind und in einem Kopplungselement 11 zusammengeführt sind (siehe Figur 4). Das Kopplungselement 11 kann beispielsweise ein elektrolytisch korrodierbares System sein, das es erlaubt, die Stentstruktur 1 vom Führungsdraht 2 abzulösen (siehe Figur 4). In der Variante gemäß Figur 2 können zwei Ablöseelemente 6, 6' zur elektrolytischen Ablösung vorgesehen sein.

Beiden Ausführungsformen ist gemein, dass der Schlitz 7 von dem Bügel 9 überbrückt wird. Der Bügel 9 selbst setzt an den Rändern 10, 10' des Maschenkonstrukts liegenden Waben an und weist mit seinem Bogen zur distalen Seite der Stentstruktur. Dies erlaubt das problemlose Einziehen der Stentstruktur in einen Katheter. Mit den angrenzenden Verbindern 5 und 5' bildet der Spannbogen 9 eine im Kopplungselement 11 (Figur 4) zusammenlaufende Fangschlaufe bzw. Öffnung eines Fangkorbs. Hierzu kann das distale Ende der Stentstruktur ebenfalls mit einer Maschenstruktur verschlossen sein.

In den Darstellungen von Figur 2 und 4, die die räumliche Wiedergabe der Stentstrukturen von Figur 1 und 3 sind, sind die auf der Rückseite liegenden Stege der Stentstruktur hell dargestellt. Zu erkennen ist der am proximalen Ende der Struktur unter dem Spannbogen 9 durchlaufende Schlitz 7, der sich zur rechten Seite hin um die Mantelfläche 8 der Stentstruktur windet. Der Schlitz 7 endet distal auf der Unterseite der Stentstruktur 1 und beschreibt damit eine Drehung um etwa 180°.

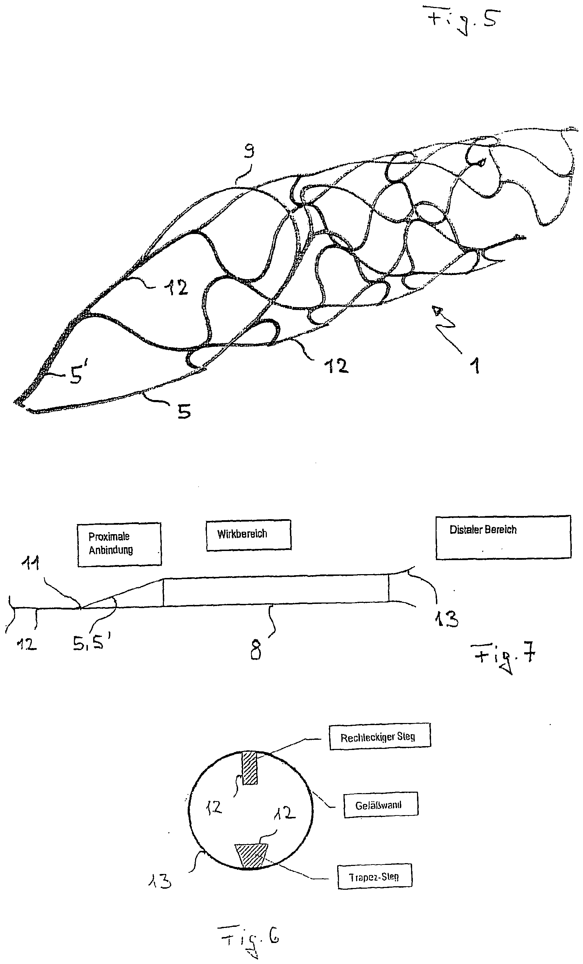

Figur 5 zeigt die räumliche Darstellung einer erfindungsgemäßen Stentstruktur, wobei die Verbinder 5 und 5' mit einwärts gerichteten Haken versehen sind, die in eine entsprechende Aufnahme eines Kopplungselements 11 eines Führungsdrahts 2 eingreifen. Solange sich das Kopplungselement mit dem proximalen Ende der Verbinder 5 und 5' in einem Katheter befindet, ist die Stentstruktur 1 an den Führungsdraht gekoppelt. Bei Herausschieben aus dem Katheter erlischt die Verbindung zwischen den Verbindern 5, 5' und dem Kopplungselement 11 und die Struktur ist als Stent zum Verbleib im Gefäßsystem freigesetzt. Die Abkopplung wird aber nur in besonderen (Not)Fällen stattfinden, etwa wenn die Vorrichtung nicht ohne weiteres wieder in den Katheter zurückgezogen werden kann.

Deutlich zu erkennen in Figur 5 ist die Schlaufenstruktur aus Bogen 9 und Verbindern 5, 5' und der Verlauf der Stege 12 der Stentstruktur entlang der Mantelfläche 8, die mit ihrem Kanten dazu dienen, auf das zu entfernende Thrombusmaterial einzuwirken und dieses von der Gefäßwand abzuscheren.

Figur 6 zeigt die beiden bevorzugten Ausführungsformen von den Stegen 12 mit rechteckigem und trapezförmigem Querschnitt, wobei die schmale Seite jeweils zur Mantelfläche 8 der Stentstruktur 1 bzw. zur Gefäßwand 13 zeigt. Diese Ausführungsvarianten gewährleisten die notwendige Stabilität des Maschennetzes einerseits und eine gute Scher- und Verdrängungswirkung auf den Thrombus andererseits.

Figur 7 zeigt schematisch den Aufbau einer erfindungsgemäßem Thrombektomievorrichtung mit dem Führungsdraht 2 dem Kopplungselement 11, dem Bereich der proximalen Anbindung mit den Verbindern 5, 5', dem Wirkbereich mit der Mantelfläche 8 und dem distalen Bereich 13 mit einer trompetenförmigen Erweiterung.

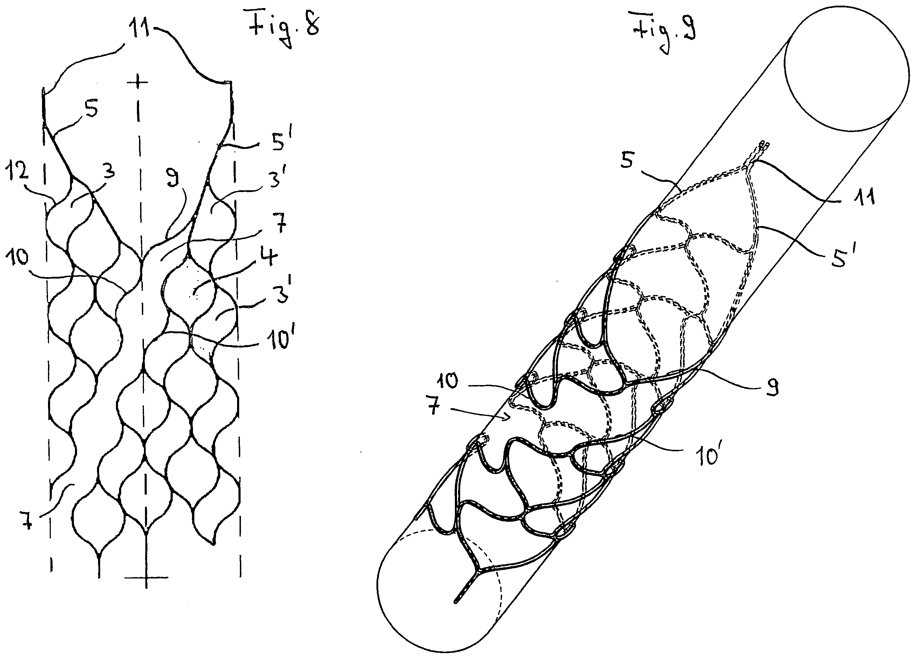

Figur 8 zeigt eine weitere Ausführungsform einer erfindungsgemäßen Thrombektomievorrichtung, die in wesentlichen Punkten mit der entsprechenden Vorrichtung gemäß Figur 1 übereinstimmt. Dieser gegenüber verändert ist die Ausführung des Spannbügels 9, der den Schlitz 7 proximal überspannt bzw. überbrückt. Dabei nimmt der Spannbügel 9 den Verlauf der Seitenfläche bzw. des Randes der Maschenstruktur 10 mit seinem wellenförmigen Verlauf auf und setzt diesen zum gegenüberliegenden Rand 10' fort. Die Verbinder 5, 5' mit den daran anschließenden Maschenrändern und der Spannbügel 9 bilden insgesamt eine Art Schlaufe, ähnlich der Öffnung eines Fangkorbs, der das Einziehen der Thrombektomievorrichtung in einen Katheter erleichtert und gleichzeitig geeignet ist, an Gefäßwänden anhängende Thromben oder Thrombenreste abzuscheren.

Es versteht sich, dass Figur 8, wie auch Figur 1 und 2, die erfindungsgemäße Vorrichtung in aufgeschnittenem Zustand, also flächig zeigen. Tatsächlich handelt es sich um eine räumliche rohrförmige Struktur, wie in Figur 9 eingepasst in ein Rohr, gezeigt.

Die Darstellung in Figur 9 zeigt die Thrombektomievorrichtung von Figur 8 in räumlicher Darstellung, wobei die auf der Vorderseite liegenden Stege und Maschen mit ausgezogenen Linien dargestellt sind und die auf der Rückseite liegenden in gestrichelter Form. Die beiden Verbinder 5 und 5' sind im Kopplungselement 11 zusammengeführt und bilden mit den daran anschließenden Maschenrändern und dem Spannbügel 9 den vorstehend beschriebenen "Fangkorb". Zu erkennen ist der wendelartige Verlauf des Schlitzes 7. Der Schlitz 7 wird begrenzt durch die Maschenränder 10 und 10' und überbrückt durch den Spannbügel 9.

In den Abbildungen stellen gleiche Bezugszeichen gleiche Sachverhalte dar.

## Patentansprüche

1. Thrombektomievorrichtung mit
einer im Wesentlichen zylindrischen Stentstruktur (1), die eine Vielzahl von Maschen (3, 4) aufweist sowie zwei Verbinder (5, 5'), die an verschiedenen Maschen (3) am proximalen Ende der Stentstruktur (1) angeordnet sind und
einem Führungsdraht (2), der ein Kopplungselement (11) aufweist, an das die Verbinder (5, 5') angekoppelt sind,
**gekennzeichnet durch** einen Schlitz (7), der sich wendelförmig über die Mantelfläche (8) der Stentstruktur (1) erstreckt und einen Spannbügel (9), der am proximalen Ende der Stentstruktur (1) den Schlitz (7) wellenförmig überspannt.

2. Vorrichtung nach Anspruch 1, **dadurch gekennzeichnet, dass** sie aus einem Formgedächtnismaterial besteht, vorzugsweise aus Nitinol oder einer Nickel-Titan-Chrom-Legierung.

3. Vorrichtung nach Anspruch 1 oder 2, **dadurch gekennzeichnet, dass** der Spannbügel (9) mit seinem Bogen zum distalen Ende der Stentstruktur (1) weist.

4. Vorrichtung nach einem der Ansprüche 1 bis 3, **dadurch gekennzeichnet, dass** der Spannbügel (9) und die Verbinder (5, 5') eine Schlaufe bilden, die im Kopplungselement (11) zusammenläuft.

5. Vorrichtung nach einem der vorstehenden Ansprüche, **dadurch gekennzeichnet, dass** sie einen oder mehrere weitere Bügel (9) im zentralen und/oder distalen Teil der Stentstruktur (1) aufweist.

6. Vorrichtung nach einem der vorstehenden Ansprüche, **dadurch gekennzeichnet, dass** die Stentstruktur (1) aus einem Rohr geschnitten ist und rechteckige oder trapezförmige Stegquerschnitte aufweist.

7. Vorrichtung nach Anspruch 6, **dadurch gekennzeichnet, dass** die Stegquerschnitte mit ihrer schmalen Seite die Mantelfläche (8) der Stentstruktur (1) bilden.

8. Vorrichtung nach einem der vorstehenden Ansprüche, **dadurch gekennzeichnet, dass** die Stentstruktur (1) mechanisch, hydraulisch oder elektrochemisch vom Führungsdraht (2) ablösbar ist.

9. Vorrichtung nach Anspruch 8, **dadurch gekennzeichnet, dass** das Kopplungselement (11) als Ablöseelement ausgebildet ist.

10. Vorrichtung nach Anspruch 8, **gekennzeichnet durch** zwei Ablösestellen, vorzugsweise mit elektrochemischer Ablösung.

11. Vorrichtung nach einem der vorstehenden Ansprüche, **dadurch gekennzeichnet, dass** das Kopplungselement (11) peripher angeordnet ist.

12. Vorrichtung nach einem der vorstehenden Ansprüche, **dadurch gekennzeichnet, dass** das distale Ende der Stentstruktur (1) kegelig oder trompetenförmig aufgeweitet ist.

13. Vorrichtung nach einem der vorstehenden Ansprüche, **gekennzeichnet durch** Markerelemente.

## Claims

1. Thrombectomy device with
an essentially cylindrical stent structure (1) having a plurality of meshes (3, 4) as well as two connectors (5, 5') arranged at different meshes (3) at the proximal end of the stent structure (1), and
a guidewire (2) provided with a coupling element (11) to which the connectors (5, 5') are attached,
**characterized by** a slot (7) extending in a helical or coiled fashion over the generated surface (8) of the stent structure (1), and a retaining clip (9) spanning the slot (7) in a wave-like manner at the proximal end of the stent structure (1).

2. Device according to claim 1, **characterized in that** it consists of a shape memory material, preferably of Nitinol or a nickel-titanium-chromium alloy.

3. Device according to claim 1 or 2, **characterized in that** the curvature of the retaining clip (9) points towards the distal end of the stent structure (1).

4. Device according to any one of claims 1 to 3, **characterized in that** the retaining clip (9) and the connectors (5, 5') form a loop that terminates in coupling element (11).

5. Device according to any one of the above claims, **characterized in that** said device is provided with one or several additional retaining clips (9) arranged in the central and/or distal portion of the stent structure (1).

6. Device according to any one of the above claims, **characterized in that** the stent structure (1) is cut out of a tube and provided with strands having rectangular or trapezoidal cross sections.

7. Device according to claim 6, **characterized in that** the generated surface (8) of the stent structure (1) is formed by the narrow or small side of the strand cross sections.

8. Device according to any one of the above claims, **characterized in that** the stent structure (1) can be detached from the guidewire (2) mechanically, hydraulically, or electrochemically.

9. Device according to claim 8, **characterized in that** the coupling element (11) is designed as detachment or severance element.

10. Device according to claim 8, **characterized by** two detachment locations, preferably having electrochemical detachment characteristics.

11. Device according to any one of the above claims, **characterized in that** the coupling element (11) is arranged peripherally.

12. Device according to any one of the above claims, **characterized in that** the distal end of the stent structure (1) has a cone- or trumpet-shaped enlargement.

13. Device according to any one of the above claims, **characterized by** marker elements.

## Revendications

1. Dispositif de thrombectomie, avec
une structure d'endoprothèse essentiellement cylindrique (1), qui présente une multiplicité de mailles (3, 4) ainsi que deux connecteurs (5, 5'), qui sont disposés à différentes mailles (3) à l'extrémité proximale de la structure d'endoprothèse (1) et
un fil de guidage (2), qui présente un élément de couplage (11), auquel les connecteurs (5, 5') sont couplés,
**caractérisé par** une fente (7), qui s'étend en hélice sur la face latérale (8) de la structure d'endoprothèse (1) et un étrier de serrage (9), qui enjambe en forme ondulée la fente (7) à l'extrémité proximale de la structure d'endoprothèse (1).

2. Dispositif selon la revendication 1, **caractérisé en ce qu'**il se compose d'un matériau à mémoire de forme, de préférence de Nitinol ou d'un alliage nickel-titane-chrome.

3. Dispositif selon la revendication 1 ou 2, **caractérisé en ce que** l'étrier de serrage (9) pointe avec son arc vers l'extrémité distale de la structure d'endoprothèse (1).

4. Dispositif selon l'une quelconque des revendications 1 à 3, **caractérisé en ce que** l'étrier de serrage (9) et les connecteurs (5, 5') forment une boucle, qui se rejoint dans l'élément de couplage (11).

5. Dispositif selon l'une quelconque des revendications précédentes, **caractérisé en ce qu'**il présente un ou plusieurs autre(s) étrier(s) (9) dans la partie centrale et/ou distale de la structure d'endoprothèse (1).

6. Dispositif selon l'une quelconque des revendications précédentes, **caractérisé en ce que** la structure d'endoprothèse (1) est découpée hors d'un tube et présente une section transversale des nervures rectangulaire ou trapézoïdale.

7. Dispositif selon la revendication 6, **caractérisé en ce que** les sections transversales des nervures forment avec leur côté étroit la face latérale (8) de la structure d'endoprothèse (1).

8. Dispositif selon l'une quelconque des revendications précédentes, **caractérisé en ce que** la structure d'endoprothèse (1) peut être séparée du fil de guidage (2) de façon mécanique, hydraulique ou électrochimique.

9. Dispositif selon la revendication 8, **caractérisé en ce que** l'élément de couplage (11) est réalisé sous forme d'élément de séparation.

10. Dispositif selon la revendication 8, **caractérisé par** deux points de séparation, de préférence avec séparation électrochimique.

11. Dispositif selon l'une quelconque des revendications précédentes, **caractérisé en ce que** l'élément de couplage (11) est disposé en périphérie.

12. Dispositif selon l'une quelconque des revendications précédentes, **caractérisé en ce que** l'extrémité distale de la structure d'endoprothèse (1) est évasée en forme de cône ou de trompette.

13. Dispositif selon l'une quelconque des revendications précédentes, **caractérisé par** des éléments de marquage.
